# EUROPEAN PATENT APPLICATION

(11) **EP 4 843 663 A1**
(43) Date of publication of application: **25.11.2026**
(21) Application number: 24918601.6
(22) Date of filing: 20.08.2024
(51) Int. Cl.: B01J 8/02, C07C 1/04, C07C 1/12, C07C 9/04, C10L 3/08

(54) **REACTION APPARATUS**

(30) Priority: 16.01.2024 JP 2024004457
(71) Applicant: Niterra Co., Ltd., Nagoya-shi, Aichi 461-0005 (JP)
(72) Inventor: IWASA, Shoto, Nagoya-shi, Aichi 461-0005 (JP); YABUHANA, Masaki, Nagoya-shi, Aichi 461-0005 (JP); MIZUNO, Akihito, Nagoya-shi, Aichi 461-0005 (JP)
(74) Representative: Grünecker Patent- und Rechtsanwälte PartG mbB
(86) International application number: PCT/JP2024/029529
(87) International publication number: WO 2025/154313

(57) **Abstract**

Provided is a reaction apparatus capable of reducing the amount of heat discarded to the outside of the system. A reaction apparatus (10) includes: a reactor (11) inside of which an input gas flows from an inlet toward an outlet; and a catalyst (18) disposed in the inside of the reactor, the reaction apparatus being configured to obtain a product gas by a chemical reaction occurring in the inside of the reactor, the chemical reaction involving heat generation, wherein the reactor includes a first reactor (12), and a second reactor (15) disposed downstream of the first reactor, and the reaction apparatus includes a heat exchanger (20) configured to transfer heat from the product gas to the input gas.

## Description

### TECHNICAL FIELD

The present invention relates to a reaction apparatus that obtains a product gas by a chemical reaction involving heat generation.

### BACKGROUND ART

Patent Document 1 discloses a prior art technique relating to a reaction apparatus that causes an input gas to flow through a reactor having a catalyst disposed inside, and obtains a product gas by a chemical reaction involving heat generation, wherein the reactor is provided with fins or a pipe for cooling water in order to cool a portion of the reactor.

### PRIOR ART DOCUMENT

### PATENT DOCUMENT

Patent Document 1: Japanese Patent Application Laid-Open (kokai) No. 2000-243425

### SUMMARY OF THE INVENTION

### PROBLEM TO BE SOLVED BY THE INVENTION

The prior art technique has the problem that the heat generated by the chemical reaction is removed by the cooling water or the fins and discarded to the outside of the system of the reaction apparatus.

The present invention has been made to solve this problem, and it is an object of the present invention to provide a reaction apparatus capable of reducing the amount of heat discarded to the outside of the system.

### MEANS FOR SOLVING THE PROBLEM

A first aspect for achieving the object is directed to a reaction apparatus including: a reactor inside of which an input gas flows from an inlet toward an outlet; and a catalyst disposed in the inside of the reactor, the reaction apparatus being configured to obtain a product gas by a chemical reaction occurring in the inside of the reactor, the chemical reaction involving heat generation, wherein the reactor includes a first reactor, and a second reactor disposed downstream of the first reactor, and the reaction apparatus includes a heat exchanger configured to transfer heat from the product gas to the input gas.

A second aspect is directed to the reaction apparatus according to the first aspect, further including a first cooler connected between the first reactor and the second reactor and configured to cool the product gas, wherein the heat exchanger includes a first heat exchanger configured to transfer heat from the product gas before the product gas enters the first cooler after exiting from the first reactor, to the input gas before the input gas enters the second reactor.

A third aspect is directed to the reaction apparatus according to the first or second aspect, wherein the heat exchanger includes a second heat exchanger configured to transfer heat from the product gas before the product gas enters the first cooler after exiting from the first reactor, to the input gas before the input gas enters the first reactor.

A fourth aspect is directed to the reaction apparatus according to any one of the first to third aspects, further including: a second heater configured to heat the second reactor; and a second cooler connected downstream of the second reactor and configured to cool the product gas, wherein the heat exchanger includes a third heat exchanger configured to transfer heat from the product gas before the product gas enters the second cooler after exiting from the second reactor, to the input gas before the input gas enters the first reactor.

A fifth aspect is directed to the reaction apparatus according to any one of the first to fourth aspects, further including a first heater configured to heat the first reactor.

A sixth aspect is directed to the reaction apparatus according to any one of the first to fifth aspects, wherein the chemical reaction is a reaction of synthesizing methane from a gas including hydrogen and at least one of carbon dioxide and carbon monoxide.

### ADVANTAGEOUS EFFECTS OF THE INVENTION

According to the present invention, since the heat of the product gas is transferred to the input gas by the heat exchanger, the product gas is cooled, and the input gas is heated. Since the input gas heated by the product gas enters the reactor to cause an exothermic reaction, it is possible to reduce the amount of heat discarded to the outside of the system.

### BRIEF DESCRIPTION OF THE DRAWINGS

[FIG. 1] FIG. 1 is a piping system diagram of a reaction apparatus according to a first embodiment.
[FIG. 2] FIG. 2 is a piping system diagram of a reaction apparatus according to a second embodiment.
[FIG. 3] FIG. 3 is a piping system diagram of a reaction apparatus according to a third embodiment.

### MODES FOR CARRYING OUT THE INVENTION

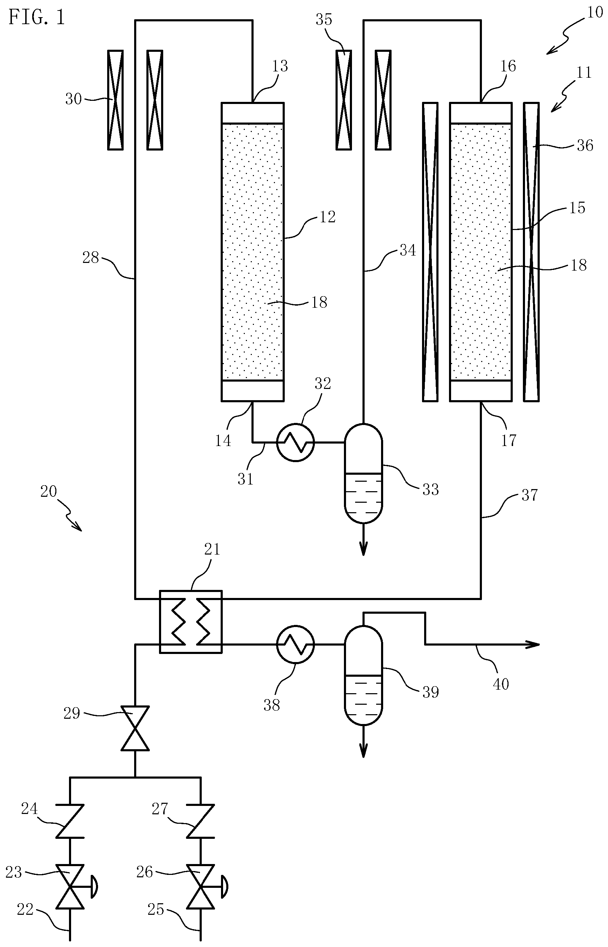

Preferred embodiments of the present invention will be described below with reference to the accompanying drawings. FIG. 1 is a piping system diagram of a reaction apparatus 10 according to a first embodiment. The reaction apparatus 10 includes a reactor 11 through which an input gas flows and that obtains a product gas by an exothermic reaction, and a heat exchanger 20 that transfers heat from the product gas to the input gas.

The reactor 11 includes a first reactor 12, and a second reactor 15 disposed downstream of the first reactor 12. The input gas enters the first reactor 12 from an inlet 13 and flows through the first reactor 12. The input gas enters the second reactor 15 from an inlet 16 and flows through the second reactor 15.

A catalyst 18 is disposed in the reactor 11. The catalyst 18 lowers the activation energy of a chemical reaction of the input gas, thereby facilitating the progress of the chemical reaction. A product gas containing a product of a chemical reaction generated in the first reactor 12 exits from an outlet 14 of the first reactor 12. A product gas containing a product of a chemical reaction generated in the second reactor 15 exits from an outlet 17 of the second reactor 15.

Any catalysts suitable for various chemical reactions may be used as the catalyst 18 without limitation. Examples of the catalysts 18 include a catalyst in the form of powders, a catalyst in the form of pellets, and a catalyst in the form of a porous structure, in each of which particles are supported on a carrier. Examples of the carrier include powders, pellets, and a porous structure made of an oxide including one or more of alumina, silica, magnesia, titania, zirconia, niobia, silica-alumina, zeolite, and calcium phosphate. The porous structure has gas permeability that allows the input gas to pass therethrough. Also, the input gas passes through voids between powders and pellets. Examples of the particles supported on the carrier include metal particles including one or more of Fe, Co, Ni, Cu, Ru, Rh, Pd, Ag, Ir, Pt, and Au.

There is no limitation on the distribution of catalytic activity in the reactor 11. For example, the catalyst 18 may be disposed such that the catalytic activity is substantially uniform over the entire length in a direction in which the gas flows, or the catalyst 18 may be disposed such that the catalytic activity on the upstream side is lower than the catalytic activity on the downstream side. If the carrier and the material and particle size of the particles are the same, the catalytic activity is proportional to the surface area of the particles supported on the carrier. Therefore, the catalytic activity of the catalyst 18 can be lowered by reducing the surface area of the particles supported on the carrier. The catalytic activity can also be lowered by mixing inactive particles having no catalytic activity into the catalyst 18 to reduce the amount of the catalyst 18 contained in a certain amount of the mixture. Examples of the inactive particles include powders and pellets made of an oxide including one or more of alumina, silica, magnesia, titania, zirconia, niobia, silica-alumina, zeolite, and calcium phosphate.

Two pipes 22 and 25 are connected to the reactor 11. The pipe 22 is a pipe to which a first source gas is supplied, and a control valve 23 and a check valve 24 are disposed thereon in this order from the upstream side toward the downstream side. The pipe 25 is a pipe to which a second source gas is supplied, and a control valve 26 and a check valve 27 are disposed thereon in this order from the upstream side toward the downstream side. A gate valve 29 is disposed on a gas pipe 28 into which the pipes 22 and 25 merge. The first and second source gases pass through the control valves 23 and 26, respectively, and thus are mixed at an optimum mixing ratio. A gas mixture (input gas) produced as a result of mixing of the two source gases flows through the gas pipe 28 via the gate valve 29.

In the present embodiment, a case will be described in which the first source gas is hydrogen and the second source gas is at least one of carbon dioxide and carbon monoxide. The reactor 11 is set to a proper pressure and performs methane production (methanation) represented by a chemical reaction formula CO₂ + 4H₂ → CH₄ + 2H₂O or CO + 3H₂ → CH₄ + H₂O.

Methanation is one example of the chemical reaction that occurs in the reactor 11, and the chemical reaction is not limited thereto. For example, the following chemical reactions can be caused to occur in the reactor 11 by appropriately selecting the type of the source gas, the type of the catalyst 18, and reaction conditions.

Synthesis gas production by partial oxidation of methane: 2CH₄ + O₂ → 2CO + 4H₂
Methanol synthesis: CO + 2H₂ → CH₃OH
Methanol synthesis: CO₂ + 3H₂ → CH₃OH + H₂O
Fischer-Tropsch synthesis: CO + 2H₂→ -(CH₂)- + H₂O
where -(CH₂)- represents a linear hydrocarbon
Dimethyl ether synthesis: 2CO + 4H₂ → CH₃OCH₃ + H₂O
Ammonia synthesis: N₂ + 3H₂ → 2NH₃

The heat exchanger 20 includes a third heat exchanger 21. The gas pipe 28 is connected to the inlet 13 of the first reactor 12 and guides the input gas to the first reactor 12. On the gas pipe 28, downstream of the gate valve 29, the third heat exchanger 21 and a first heater 30 are disposed in this order in the flow direction of the input gas. The third heat exchanger 21 transfers heat from the product gas of the second reactor 15 to the input gas to the first reactor 12. The third heat exchanger 21 performs heat exchange between the low-temperature input gas flowing through the gas pipe 28 and the high-temperature product gas flowing through a gas pipe 37 (which will be described later). There is no limitation on the third heat exchanger 21, but a plate-type heat exchanger is preferable because of its high coefficient of heat transfer. In the plate-type heat exchanger, heat exchange is performed by causing the input gas and the product gas to alternately flow between stacked heat transfer plates.

The first heater 30 heats the input gas to the first reactor 12. Examples of the first heater 30 include a heater that heats the gas pipe 28 by heating a heat source by electricity or combustion heat of a gas, and a heater that utilizes induction heating. In particular, the first heater 30 that heats the heat source by electricity is preferable because the accuracy of temperature control is high. If the input gas to the first reactor 12 has a temperature equal to or higher than the lowest temperature at which the exothermic reaction starts, the exothermic reaction proceeds in the first reactor 12 without heating the first reactor 12 using a heater.

The product gas of the first reactor 12 flows through a gas pipe 31 connected to the outlet 14 of the first reactor 12. A first cooler 32 and a first separator 33 are disposed on the gas pipe 31 in this order in the flow direction of the product gas. The first cooler 32 cools the product gas of the first reactor 12 to a temperature equal to or lower than the dew point, thereby condensing the water vapor contained in the product gas. The first separator 33 separates the condensed water and the gas.

A gas pipe 34 connects the first separator 33 and the inlet 16 of the second reactor 15. A third heater 35 is disposed on the gas pipe 34 through which the input gas to the second reactor 15 flows. The third heater 35 heats the input gas to the second reactor 15, for example, to a temperature equal to or higher than the lowest temperature at which the exothermic reaction starts. For the third heater 35, the same mechanism as that of the first heater 30 is employed.

A second heater 36 is disposed in the second reactor 15. The second heater 36 is disposed between the inlet 16 and an intermediate position of the entire length of a portion where the catalyst 18 is disposed, and between the outlet 17 and the intermediate position of the entire length of the portion where the catalyst 18 is disposed. The second heater 36 secures the reaction rate in the second reactor 15 where a large amount of chemical reaction products are present. Examples of the second heater 36 include a heater that heats the catalyst 18 by heating a heat source by electricity or combustion heat of a gas, and a heater that utilizes induction heating. In particular, the second heater 36 that heats the heat source by electricity is preferable because the accuracy of temperature control is high.

The product gas of the second reactor 15 flows through a gas pipe 37 connected to the outlet 17 of the second reactor 15. The second heater 36 heats the catalyst 18 such that the temperature of the product gas entering the gas pipe 37 from the outlet 17 falls within a predetermined range (e.g., ±10°C). The quality of the product gas can be stabilized by controlling the temperature of the catalyst 18 by the second heater 36.

The third heat exchanger 21, a second cooler 38, and a second separator 39 are disposed on the gas pipe 37 in this order in the flow direction of the product gas. The third heat exchanger 21 transfers the heat of the product gas flowing through the gas pipe 37 to the input gas flowing through the gas pipe 28, thereby heating the input gas. The second cooler 38 cools the product gas of the second reactor 15 to a temperature equal to or lower than the dew point, thereby condensing the water vapor contained in the product gas. The second separator 39 separates the condensed water and the gas. The product (gas) from which water has been separated flows through a gas pipe 40 connected to the second separator 39.

As a result of a chemical reaction occurring while the input gas flows through the catalyst 18 in the first reactor 12, the product gas of the first reactor 12 contains methane, water vapor, and the unreacted input gas. Since the chemical reaction is an exothermic reaction, the temperature of the product gas of the first reactor 12 is higher than the temperature of the input gas to the first reactor 12. The product gas of the first reactor 12 is cooled to a temperature equal to or lower than the dew point by the first cooler 32, and the first separator 33 separates the condensed water. Accordingly, the amount of the water vapor contained in the product gas of the first reactor 12 (the input gas to the second reactor 15) is reduced, thereby shifting the equilibrium of the chemical reaction in the second reactor 15 to the right. Therefore, the progress of the reaction of the unreacted input gas is facilitated in the second reactor 15.

It is preferable that the input gas to the second reactor 15 cooled by the first cooler 32 is heated by the third heater 35 to a temperature equal to or higher than the lowest temperature at which the exothermic reaction starts, because this facilitates the progress of the chemical reaction in the second reactor 15. Since the second reactor 15 is heated by the second heater 36, the reaction rate in the second reactor 15 is increased, whereby the reaction conversion is improved.

Since the second reactor 15 is heated by the second heater 36, the thermal energy of the product gas of the second reactor 15 heated by the second heater 36 is greater than the thermal energy of the input gas to the second reactor 15. The thermal energy of the product gas of the second reactor 15 before the product gas enters the second cooler 38 after exiting from the second reactor 15 is transferred by the third heat exchanger 21 to the input gas to the first reactor 12. Accordingly, it is possible to reduce the amount of heat of the product gas of the second reactor 15 that is cooled by the second cooler 38 and discarded to the outside of the system. Furthermore, the product gas of the second reactor 15 is cooled by the third heat exchanger 21. Accordingly, it is possible to reduce the amount of energy to be input to the second cooler 38 in order to cool the product gas of the second reactor 15.

It is possible to omit the first heater 30 if the temperature of the input gas to the first reactor 12 is increased by the third heat exchanger 21 to a temperature equal to or higher than the lowest temperature at which the exothermic reaction starts in the first reactor 12. When the first heater 30 is omitted, the increment in thermal energy of the input gas to the first reactor 12 depends on the thermal energy of the product gas of the second reactor 15 in the third heat exchanger 21. Even when the first heater 30 is not omitted, since the input gas to the first reactor 12 is heated by the third heat exchanger 21, it is possible to reduce the amount of energy to be input to the first heater 30 in order to heat the input gas to the first reactor 12.

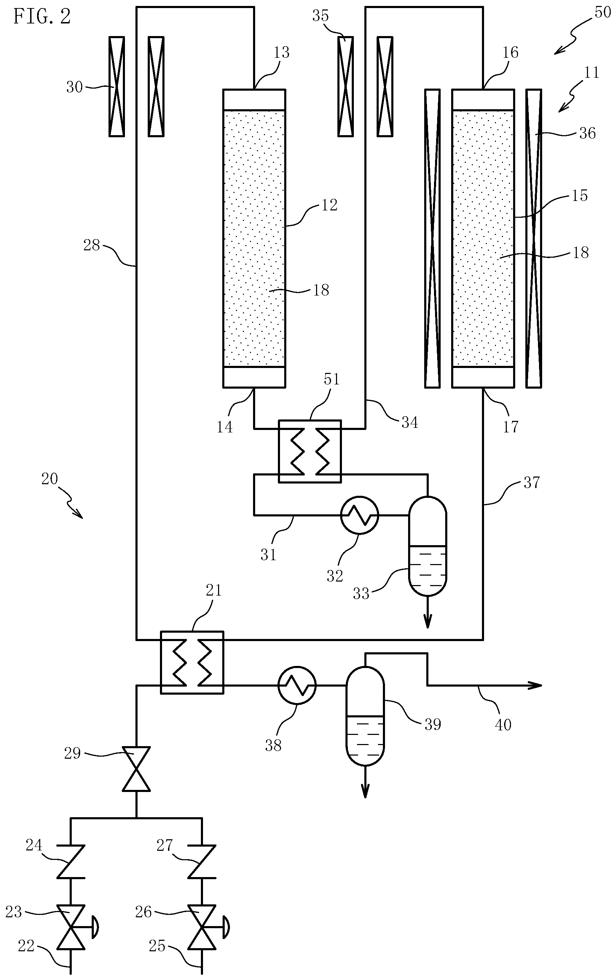

With reference to FIG. 2, a reaction apparatus 50 according to a second embodiment will be described. In the first embodiment, a case has been described in which the heat exchanger 20 includes the third heat exchanger 21. In contrast, in the second embodiment, a case will be described in which the heat exchanger 20 includes the third heat exchanger 21 and a first heat exchanger 51. Portions that are the same as the portions described in the first embodiment are denoted by the same reference characters, and the description thereof is omitted.

FIG. 2 is a piping system diagram of the reaction apparatus 50 according to the second embodiment. The reaction apparatus 50 includes a first heat exchanger 51. The first heat exchanger 51 is disposed between the first reactor 12 and the first cooler 32 on the gas pipe 31, and between the first separator 33 and the third heater 35 on the gas pipe 34. The first heat exchanger 51 transfers, to the input gas to the second reactor 15, the heat of the product gas of the first reactor 12 before the product gas enters the first cooler 32 after exiting from the first reactor 12. Since the input gas to the second reactor 15 is heated by the first heat exchanger 51, it is possible to reduce the amount of energy to be input to the third heater 35 in order to heat the input gas to the second reactor 15.

It is possible to omit the third heater 35 if the temperature of the input gas to the second reactor 15 is increased by the first heat exchanger 51 to a temperature equal to or higher than the lowest temperature at which the exothermic reaction starts in the second reactor 15. When the third heater 35 is omitted, the increment in thermal energy of the input gas to the second reactor 15 depends on the thermal energy of the product gas of the first reactor 12 in the first heat exchanger 51.

The thermal energy of the product gas of the first reactor 12 before the product gas enters the first cooler 32 after exiting from the first reactor 12 is transferred to the input gas to the second reactor 15 by the first heat exchanger 51. Accordingly, it is possible to reduce the amount of heat of the product gas of the first reactor 12 that is cooled by the first cooler 32 and discarded to the outside of the system. Furthermore, the product gas of the first reactor 12 is cooled by the first heat exchanger 51. Accordingly, it is possible to reduce the amount of energy to be input to the first cooler 32 in order to cool the product gas of the first reactor 12.

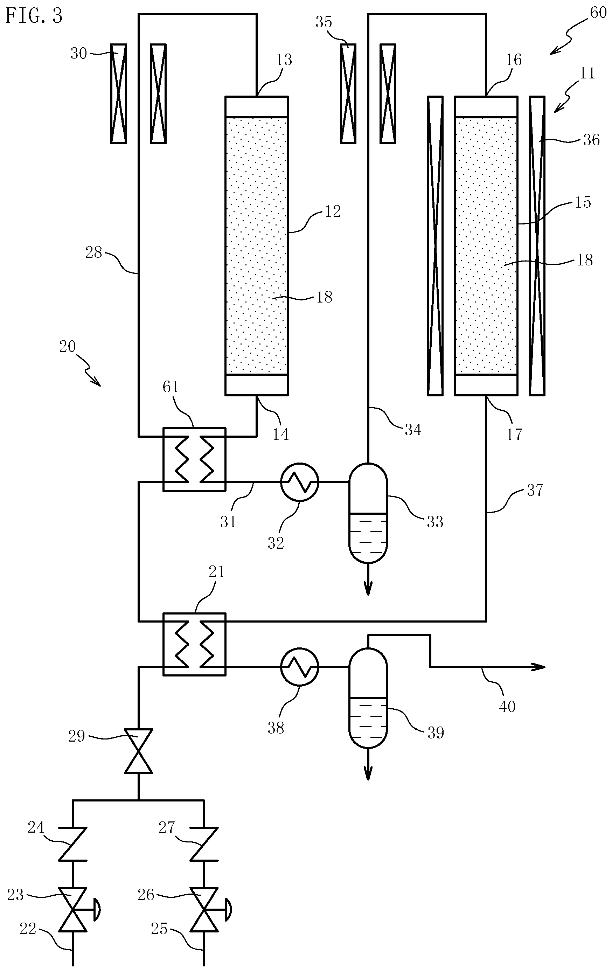

With reference to FIG. 3, a reaction apparatus 60 according to a third embodiment will be described. In the second embodiment, a case has been described in which the heat exchanger 20 includes the third heat exchanger 21 and the first heat exchanger 51. In contrast, in the third embodiment, a case will be described in which the heat exchanger 20 includes the third heat exchanger 21 and a second heat exchanger 61. Portions that are the same as the portions described in the first embodiment are denoted by the same reference characters, and the description thereof is omitted.

FIG. 3 is a piping system diagram of the reaction apparatus 60 according to the third embodiment. The reaction apparatus 60 includes a second heat exchanger 61. The second heat exchanger 61 is disposed between the first reactor 12 and the first cooler 32 on the gas pipe 31, and between the third heat exchanger 21 and the first heater 30 on the gas pipe 28. The second heat exchanger 61 transfers, to the input gas to the first reactor 12, the heat of the product gas of the first reactor 12 before the product gas enters the first cooler 32 after exiting from the first reactor 12.

The thermal energy of the product gas of the first reactor 12 before the product gas enters the first cooler 32 after exiting from the first reactor 12 is transferred to the input gas to the first reactor 12 by the second heat exchanger 61. Accordingly, it is possible to reduce the amount of heat of the product gas of the first reactor 12 that is cooled by the first cooler 32 and discarded to the outside of the system. Furthermore, the product gas of the first reactor 12 is cooled by the second heat exchanger 61. Accordingly, it is possible to reduce the amount of energy to be input to the first cooler 32 in order to cool the product gas of the first reactor 12.

Since the input gas to the first reactor 12 is heated by the second heat exchanger 61, it is possible to reduce the amount of energy consumed by the first heater 30 that heats the input gas to the first reactor 12. It is possible to omit the first heater 30 if the temperature of the input gas to the first reactor 12 is increased by the second heat exchanger 61 to a temperature equal to or higher than the lowest temperature at which the exothermic reaction starts in the first reactor 12. When the first heater 30 is omitted, the increment in thermal energy of the input gas to the first reactor 12 depends on the thermal energy of the product gas of the second reactor 15 in the third heat exchanger 21, and the thermal energy of the product gas of the first reactor 12 in the second heat exchanger 61.

Although the present invention has been described based on the embodiments, the present invention is not limited to the above-described embodiments at all. It can be easily understood that various improvements and modifications can be made without departing from the gist of the present invention.

In the embodiments, a case has been described in which the reactor 11 includes the first reactor 12 and the second reactor 15. However, the present invention is not necessarily limited thereto. It is, of course, possible that one or more reactors are further connected downstream of the second reactor 15, or one or more reactors are further connected upstream of the first reactor 12.

In the embodiments, a case has been described in which a heater that heats the first reactor 12 is not disposed in the first reactor 12. However, the present invention is not necessarily limited thereto. It is, of course, possible that a heater that heats the catalyst 18 housed in the first reactor 12 is disposed in the first reactor 12.

### DESCRIPTION OF REFERENCE NUMERALS

10,50,60 reaction apparatus
11 reactor
12 first reactor
13 inlet
14 outlet
15 second reactor
16 inlet
17 outlet
18 catalyst
20 heat exchanger
21 third heat exchanger
30 first heater
32 first cooler
36 second heater
38 second cooler
51 first heat exchanger
61 second heat exchanger

## Claims

1. A reaction apparatus comprising:
a reactor inside of which an input gas flows from an inlet toward an outlet; and
a catalyst disposed in the inside of the reactor,
the reaction apparatus being configured to obtain a product gas by a chemical reaction occurring in the inside of the reactor, the chemical reaction involving heat generation, wherein
the reactor includes a first reactor, and a second reactor disposed downstream of the first reactor, and
the reaction apparatus comprises a heat exchanger configured to transfer heat from the product gas to the input gas.

2. The reaction apparatus according to claim 1, further comprising a first cooler connected between the first reactor and the second reactor and configured to cool the product gas, wherein
the heat exchanger includes a first heat exchanger configured to transfer heat from the product gas before the product gas enters the first cooler after exiting from the first reactor, to the input gas before the input gas enters the second reactor.

3. The reaction apparatus according to claim 1, further comprising a first cooler connected between the first reactor and the second reactor and configured to cool the product gas, wherein
the heat exchanger includes a second heat exchanger configured to transfer heat from the product gas before the product gas enters the first cooler after exiting from the first reactor, to the input gas before the input gas enters the first reactor.

4. The reaction apparatus according to any one of claims 1 to 3, further comprising:
a second heater configured to heat the second reactor; and
a second cooler connected downstream of the second reactor and configured to cool the product gas, wherein
the heat exchanger includes a third heat exchanger configured to transfer heat from the product gas before the product gas enters the second cooler after exiting from the second reactor, to the input gas before the input gas enters the first reactor.

5. The reaction apparatus according to claim 4, further comprising a first heater configured to heat the first reactor.

6. The reaction apparatus according to any one of claims 1 to 3, wherein the chemical reaction is a reaction of synthesizing methane from a gas including hydrogen and at least one of carbon dioxide and carbon monoxide.
